# EUROPEAN PATENT SPECIFICATION

(11) **EP 1 989 989 B1**
(45) Date of publication and mention of the grant of the patent: **26.10.2016**
(21) Application number: 08008587.1
(22) Date of filing: 07.05.2008
(51) Int. Cl.: A61B 1/00, A61B 1/05, A61B 1/015

(54) **Medical apparatus with electrical connection portions**
Medizinisches Gerät mit elektrischen Verbindungsabschnitten
Appareil médical avec des portions pour connection électrique

(30) Priority: 08.05.2007 JP 2007123854
(43) Date of publication of application: 12.11.2008
(73) Proprietor: Olympus Corporation, Tokyo 192-8507 (JP)
(72) Inventor: Miyagi, Masaaki, Hachioji-shi, Tokyo 192-8507 (JP); Takashi, Sawai, Hachioji-shi, Tokyo 192-8507 (JP)
(74) Representative: von Hellfeld, Axel

(56) References cited:
- US-A- 3 936 124
- US-A- 4 499 895
- US-A- 5 810 620

## Description

The present invention relates to a medical apparatus comprising an endoscope body, which is located, for example, on the proximal end side of an operation section of an endoscope.

In an endoscope serving as a medical apparatus, a body unit is provided at a proximal end portion of an insertion section which is inserted in a body cavity. A universal cord, which is connected, for example, to a light source device, is connected to the body unit. A plurality of built-in components, for instance, an optical fiber such as a light guide of an illumination optical system, a signal line which is connected to an image pick-up device such as a CCD of an observation optical system, bending operation wires and conduits for air feed, water feed or suction, are inserted in the insertion section. Further, the body unit is provided with a bending operation section for bend-operating a bending section, in which connection sections of the optical fiber, the signal line and the various conduits are provided, and various operation buttons for air feed, water feed and suction.

A motor-driven bending endoscope, which bend-operates the bending section by motor driving, has been developed. There is known a motor-driven bending endoscope in which an operation section is composed of an insertion-section-side body and a power unit to which the insertion-section-side body is detachably connected. The power unit includes an electric motor, a bend-driving mechanism which is driven by the electric motor, and a driving-side coupling which transmits a driving force of the bend-driving mechanism. The insertion-section-side body includes a driven-side coupling of a motor-driven angle mechanism which axially moves and operates bend-operation wires for bend-operating the bending section. If the insertion-section-side body is connected to the power unit, the driving-side coupling of the bend-driving mechanism is coupled to the driven-side coupling of the motor-driven angle mechanism.

Further, a signal processing circuit is built in the power unit. The signal processing circuit is provided with power-unit-side electrical contact portions which are electrically connected to peripheral devices, such as a video processor and a control box, via the universal cord. The insertion-section-side body is provided with insertion-section-side electrical contact portions which are electrically connected to, e.g. a solid image-pickup device that is provided in a distal-end structure section of the endoscope. If the insertion-section-side body is connected to the power unit, the insertion-section-side electrical contact portions are put in contact with, and electrically connected to, the power-unit-side electrical contact portions, and transmission/reception of electric signals is enabled.

On the other hand, in general, after the endoscope for medical treatment is used, the outer surface, as well as conduits built in the endoscope, is cleaned and sterilized. In the case of the above-described endoscope having the electrical contact portions that are exposed, it is necessary to completely remove moisture that adheres due to cleaning. Thereby, short-circuit of electrical contact portions due to moisture is prevented when the endoscope is re-used.

For instance, in Jpn. Pat. Appln. KOKAI Publication No. 6-133919 (patent document 1), the following structure is adopted as means for removing moisture adhering to the connector, when the connector of the endoscope apparatus is connected to a peripheral device. Specifically, a sponge is provided on a plug side having electrical contacts. Thereby, when the plug is pushed in and connected to a socket having terminals that are to be connected to the electrical contacts, even if moisture enters the plug, the moisture can be absorbed by the sponge.

Similarly, in Jpn. Pat. Appln. KOKAI Publication No. 2001-224556 (patent document 2), for instance, a socket side is provided with an electrode surface and a moisture wiping member that is formed of silicone rubber, and a plug side is provided with an electrode surface which is connected to the electrode surface of the socket side. When the plug is connected to the socket, moisture on the electrode surface is removed by the moisture wiping member.

However, the medical apparatuses disclosed in the above-described patent documents 1 and 2 have the following problems. Specifically, the moisture removing means in the medical apparatus of patent document 1 is configured such that moisture adhering to the electrode surface is absorbed by the water-absorbing member when the plug and socket are connected. Thus, if the water-absorbing member has fully absorbed moisture, the water-absorbing performance thereof would be lost.

The moisture removing means in the medical apparatus of patent document 2 is configured such that moisture adhering to the electrode surface is wiped by the moisture wiping member when the plug and socket are connected. In this case, if moisture flows down by force of gravity and the electrode surface is positioned in the direction of force of gravity, the electrode surface would be wetted again.

The present invention has been made in consideration of the above-described circumstances, and the object of the invention is to provide a medical apparatus, wherein even if moisture adheres to an electrical contact portion, the moisture is removed in the process of connecting a connection section, there is no concern that moisture that is removed or moisture that is not removed and remains on the connector section adheres to the electrical contact portion, and the reliability can be improved.

Document US-A-5810620 describes a connector for an endoscope apparatus wherein the electrical receptacle comprises a wiper for wiping off liquid moisture when an electrical plug is inserted into the electrical receptacle, thereby preventing a short-circuit.

The invention is a medical apparatus as defined in the independent claims 1 and 2.

Preferably, the removing member is an elastic member.

Preferably, the prevention member is an elastic member.

Preferably, the medical apparatus includes a motor-driven bending endoscope which bend-operates a bending section by motor driving, the motor-driven bending endoscope includes an insertion-section-side body and a power unit to which the insertion-section-side body is detachably connected, the first connector portion is provided on the power unit, and the second connector portion is provided on the insertion-section-side body.

Preferably, the insertion-section-side body includes a signal processing member which is connected to the electrical contact portions and the power unit includes a signal cable which is connected to the terminals for electrical connection.

Preferably, the power unit is configured such that the removing member and the prevention member are disposed spaced apart, with the terminals being interposed.

According to the present invention, there are provided the removing member for removing moisture and the prevention member for preventing entrance of moisture in a direction different from a direction of moisture removal by the removing member. Thereby, the invention has the advantage that even if moisture adheres to electrical contact portions, the moisture is removed in the process of connecting the connector section and the moisture does not adhere again to the electrical contact portions, and the reliability can be improved.

The invention can be more fully understood from the following detailed description when taken in conjunction with the accompanying drawings, in which:
FIG. 1 is a perspective view that shows the entire structure of a medical apparatus according to a first embodiment of the present invention;
FIG. 2 is a block diagram that schematically shows the structure of the medical apparatus according to the first embodiment;
FIG. 3 is a perspective view showing a state in which an insertion-section-side body and a power unit of the medical apparatus according to the first embodiment are not connected;
FIG. 4 is a longitudinal cross-sectional view showing a non-connected state of the insertion-section-side body and the power unit of the medical apparatus according to the first embodiment;
FIG. 5 is a longitudinal cross-sectional view showing a connected state of the insertion-section-side body and the power unit of the medical apparatus according to the first embodiment;
FIG. 6 is a side view showing a non-connected state of the insertion-section-side body and power unit of the medical apparatus according to the first embodiment, with the power unit alone being shown in cross section;
FIG. 7 is a side view showing a connected state of the insertion-section-side body and power unit of the medical apparatus, with the power unit alone being shown in cross section;
FIG. 8A is a plan view showing a flexible connector of the medical apparatus according to the first embodiment;
FIG. 8B is a perspective view showing the flexible connector of the medical apparatus according to the first embodiment;
FIG. 9 is a longitudinal cross-sectional view showing a non-connected state of an insertion-section-side body and a power unit of a medical apparatus according to a second embodiment of the present invention;
FIG. 10 is a longitudinal cross-sectional view showing a non-connected state of an insertion-section-side body and a power unit of a medical apparatus according to a third embodiment of the present invention;
FIG. 11 is a longitudinal cross-sectional view showing a connected state of the insertion-section-side body and power unit of the medical apparatus according to the third embodiment;
FIG. 12 is a longitudinal cross-sectional view showing a non-connected state of an insertion-section-side body and a power unit of a medical apparatus according to a fourth embodiment of the present invention; and
FIG. 13 is a longitudinal cross-sectional view showing a connected state of the insertion-section-side body and power unit of the medical apparatus according to the fourth embodiment.

Embodiments of the present invention will now be described with reference to the accompanying drawings.

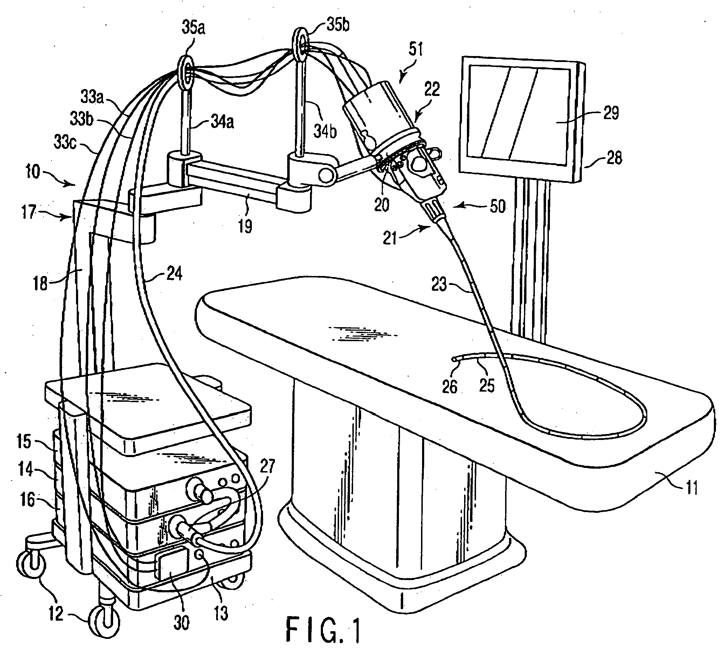

FIG. 1 to FIG. 8B show a first embodiment of the invention. FIG. 1 shows the entire structure of a medical apparatus 10. FIG. 1 is a perspective view showing a state in which the medical apparatus 10 is disposed on the side of an operation bed 11. A light source device 14, a video processor 15 and a control box 16 are mounted on the support table 13 which includes casters 12. A support column 18, which constitutes a support mechanism 17, is vertically erectly provided on the support table 13. An upper end portion of the support column 18 is provided with an endoscope holding section 20 as a medical device holding section via a plurality of arms 19 which are rotatable in a horizontal plane.

An endoscope 21 serving as a medical device is held by the endoscope holding section 20. The endoscope 21 includes a body unit 22, an elongated insertion section 23, and a universal cord 24. The body unit 22 is held by the endoscope holding section 20. A proximal end portion of the insertion section 23 is connected to the body unit 22. The insertion section 23 is provided with a bending section 25 and a distal-end structure section 26. One end portion of the universal cord 24 is connected to the body unit 22. The other end portion of the universal cord 24 is provided with a connector 27. The connector 27 is connected to the light source device 14. A light guide 115 and a signal cable (not shown), which are to be described later, are inserted in the universal cord 24.

The distal-end structure section 26 of the endoscope 21 includes, at its distal end face, an illumination lens of an illumination optical system (not shown) and an observation lens of an observation optical system (not shown). A light guide 46 of an optical fiber, which guides illumination light, is continuously inserted in the universal cord 24, body unit 22 and insertion section 23. Illumination light from the light source device 14 is emitted from the illumination lens at the front face of the distal-end structure section 26 via the light guide 46 that is inserted through the universal cord 24, body unit 22 and insertion section 23. Thereby, the inside of a body cavity is illuminated.

The observation optical system is provided with an image pick-up unit 37, such as a solid image pick-up element, at a position of a focusing plane of the observation lens. An observation image of the observation lens, which observes the inside of the body cavity, is converted to a video signal by the image pick-up unit 37. A signal cable 38, which transmits a video signal from the image pick-up unit 37, is continuously inserted through the insertion section 23, body unit 22 and universal cord 24. The video signal from the image pick-up unit 37 is transmitted to the video processor 15 via the signal cable 38. The video signal is subjected to a predetermined signal process by the video processor 15.

A control panel 28 is disposed on the operation bed 11. The control panel 28 is provided with a display unit 29 and an operation section, such as a touch-panel operation section, on a display screen of the display unit 29. The video signal that is output from the video processor 15 is transmitted to the control panel 28. A predetermined endoscopic image is displayed on the display unit 29 of the control panel 28. A surgeon can input various operational instructions from the operation section of the control panel 28.

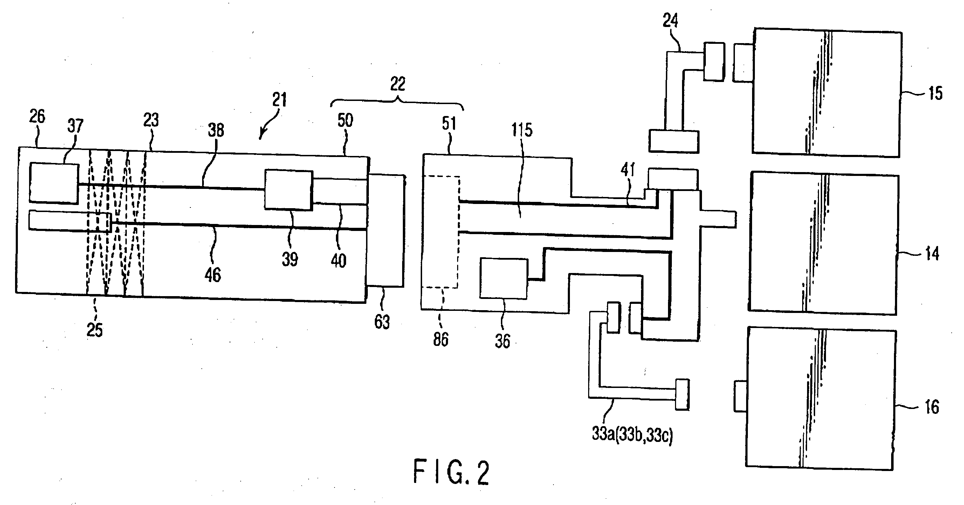

The control box 16 functions to operate an electromagnetic valve unit (not shown) for performing air feed and water feed at a time of using the endoscope 21 for observation and medical treatment. The electromagnetic valve unit executes control of air-feed/water-feed and a suction operation via an air-feed/water-feed conduit and a suction conduit, which are provided within the insertion section 23. As shown in FIG. 2, a system controller of the control box 16 is electrically connected to the light source device 14 and video processor 15.

A fluid control cassette 30 is detachably attached to the electromagnetic valve unit of the control box 16 shown in FIG. 1. The fluid control cassette 30 includes valve bodies having a flow amount adjusting mechanism, which are associated with air feed, water feed and forward water feed. The electromagnetic valve unit drives the flow amount adjusting mechanism of the fluid control cassette 30.

One end of each of three tubes 33a, 33b and 33c for performing air feed, water feed and forward water feed to the endoscope 21 is connected to the control box 16. The other end of each of the three tubes 33a, 33b and 33c is connected to the body unit 22 of the endoscope 21. These tubes 33a, 33b and 33c are formed of hollow flexible resin materials.

A plurality of support rods 34a and 34b are erectly provided on the arms 19 of the support mechanism 17. The support rods 34a and 34b are provided with retainers 35a and 35b which support intermediate portions of the tubes 33a, 33b and 33c and the universal cord 24.

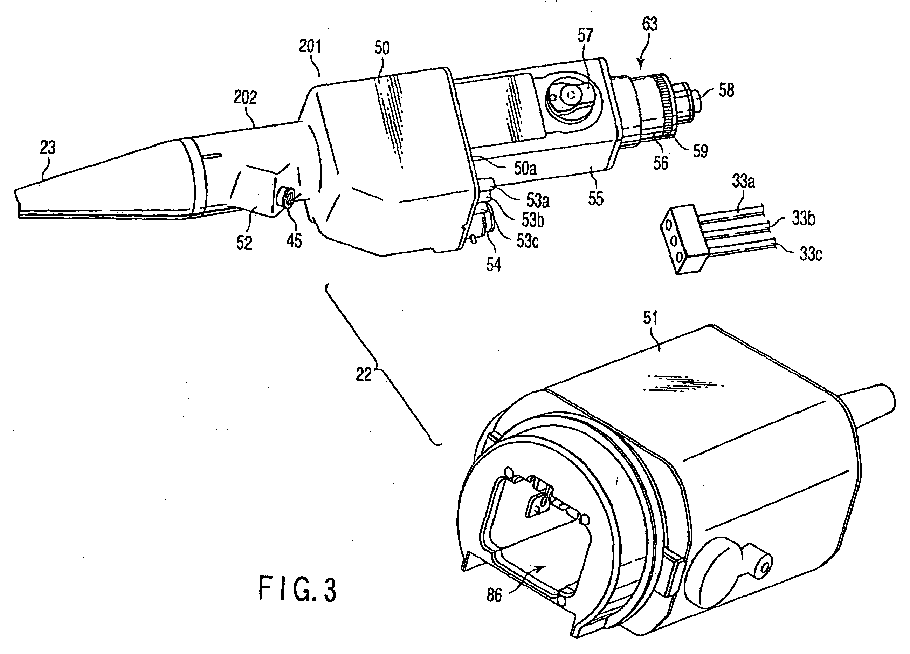

FIG. 2 is a structural view that schematically shows an internal structure of the endoscope 21. FIG. 3 is a perspective view showing the body unit 22 of the endoscope 21. The body unit 22 comprises an insertion-section-side body 50 as a first member, and a power unit 51 as a second member. The insertion-section-side body 50 is detachably connected to the power unit 51. The power unit 51 is a supply source for supplying driving force, fluid, light, electric power and a signal to the insertion-section-side body 50.

A bend-driving unit 36 including an electric motor which is controlled in accordance with a bending operation instruction, a signal cable 41, a power-unit-side fluid conduit (not shown) and a power-unit-side light guide 115 are built in the power unit 51.

A bending section 25, an observation optical system, an insertion-section-side fluid conduit (not shown), and an insertion-section-side light guide 46 are built in the insertion-section-side body 50. The distal-end structure section 26 is provided with an image pick-up unit 37 which is composed of a solid image pick-up element of the observation optical system, and an emission end portion of the light guide 46 which constitutes the illumination optical system. Further, a signal cable 38 for transmitting a video signal from the image pick-up unit 37, a video board 39, a flexible board 40 and bending operation wires (not shown) are provided in the insertion section 23.

The bend-driving mechanism 36 of the power unit 51 is configured to be connected to bending operation wires of the bending section 25 of the insertion-section-side body 50 when the power unit 51 and the insertion-section-side body 50 are coupled, and to bend-operate the bending section 25 in upward, downward, leftward and rightward directions. At the same time, the signal cable 41 of the power unit 51 is connected to the flexible board 40 of the insertion-section-side body 50, and the power-unit-side fluid conduit and power-unit-side light guide 115 are connected to the insertion-section-side fluid conduit and the insertion-section-side light guide 46. Thereby, illumination light, which is supplied from the light source device 14, is guided to the endoscope 21 via the power-unit-side light guide 115 and the insertion-section-side light guide 46, and the illumination light is emitted from the distal-end structure section 26. Further, the signal cable 38, which transmits the video signal from the image pick-up unit 37, the video board 39, the flexible board 40 and the signal cable 41 are successively connected to the video processor 15. The signal cable 41 is connected to a predetermined terminal of the video processor 15 through the inside of the body unit 22 and universal cord 24.

The external structures of the insertion-section-side body 50 and power unit 51 are described. The insertion-section-side body 50 includes a substantially box-shaped casing portion 201 and a circular cylindrical casing portion 202 having a less outside diameter than the box-shaped casing portion 201. A projection portion 52 is provided on an outer peripheral surface of the cylindrical casing portion 202. The projection portion 52 is provided with a forceps insertion hole 45. A center line 45a of the forceps insertion hole 45 is inclined outward, relative to the axial direction of the insertion-section-side body 50. Thus, even if the power unit 51 is attached to the insertion-section-side body 50, the power unit 51 does not hinder the insertion/removal of a treatment device in/from the forceps insertion hole 45.

An attachment surface 50a, which is perpendicular to the axial direction of the insertion-section-side body 50, is provided at an intermediate portion of the insertion-section-side body 50. A fluid connection connector is provided on the attachment surface 50a. The fluid connection connector includes an air-feed connector 53a, a water-feed connector 53b, a forward water-feed connector 53c and a water leak detection mouthpiece 54. The openings of these air-feed connector 53a, water-feed connector 53b, forward water-feed connector 53c and water leak detection mouthpiece 54 are disposed toward the proximal end side. In other words, the air-feed connector 53a, water-feed connector 53b and forward water-feed connector 53c are disposed on the proximal end side of the insertion-section-side body 50, that is, at positions spaced apart from the forceps insertion hole 45.

The other ends of the tubes 33a, 33b and 33c are connected to the air-feed connector 53a, water-feed connector 53b and forward water-feed connector 53c. The insertion-section-side body 50 and power unit 51 of the body unit 22 are provided with fluid conduits (not shown) which are coupled to the tubes 33a, 33b and 33c. The tubes 33a, 33b and 33c are made to communicate with the distal-end structure section 26 of the insertion section 23 via the fluid conduits. If the electromagnetic valve unit of the control box 16 is driven and an air-feed/water-feed operation is executed from the fluid control cassette 30, air-feed/water-feed can be performed from the distal-end structure section 26 via the tubes 33a and 33b.

Since the treatment device, such as a forceps or a catheter, is inserted/removed via the forceps insertion hole 45, there may be a case in which the forceps insertion hole 45 becomes an unclean area. However, since the forceps insertion hole 45 is spaced apart from the air-feed connector 53a, water-feed connector 53b and forward water-feed connector 53c, infection to parts, other than the forceps insertion hole 45, can be prevented.

An endoscope auto-cleaning device, which cleans the endoscope 21 after use, is required to automatically perform three functions, i.e. a function of water leak detection, a function of air feed/water feed to respective conduits, and a function of insertion of a brush into the respective conduits via the air-feed connector 53a, water-feed connector 53b and forward water-feed connector 53c. The air-feed connector 53a, water-feed connector 53b, forward water-feed connector 53c and water leak detection mouthpiece 54 are laterally arranged in line and the opening of the water leak detection mouthpiece 54 is disposed in parallel to the air-feed connector 53a, water-feed connector 53b and forward water-feed connector 53c. Thereby, the endoscope auto-cleaning device can perform the above-described three functions by control in one direction alone. Moreover, the water leak detection mouthpiece 54 is disposed outermost in consideration of preventing other mouthpieces from becoming obstacles when water leak detection is manually performed.

An angular cylindrical portion 55 and a circular cylindrical portion 56 are integrally provided on a proximal end portion of the insertion-section-side body 50. The circular cylindrical portion 56 is disposed at the rear end of the angular cylindrical portion 55. The above-described bend-driving mechanism 36 is built in the angular cylindrical portion 55. A driven-side coupling 57, which functions as a driven-side member that is interlocked with the bend-driving mechanism 36, is provided on an outside part of the angular cylindrical portion 55. A light guide connector 58 is provided on a rear end face of the circular cylindrical portion 56. The light guide connector 58 is a light transmission connector receiver which is connected to the light guide 115. Further, a rear end portion of the circular cylindrical portion 56 is formed of an electrically insulating material, and a great number of electrodes 59 functioning as electric signal connectors are disposed in a circumferential direction on an outer peripheral surface of the rear end portion of the circular cylindrical portion 56.

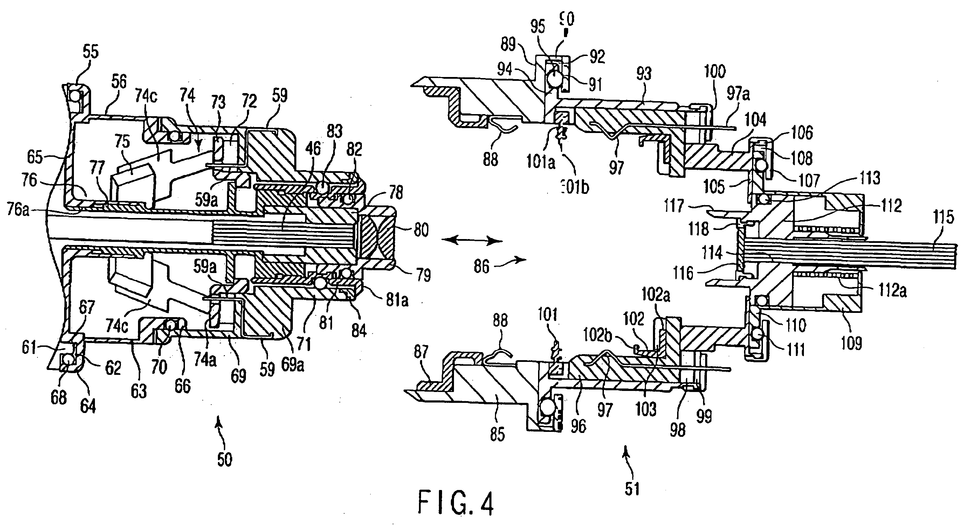

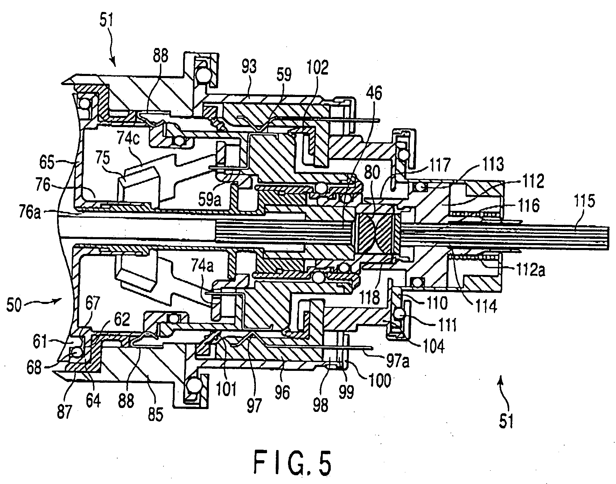

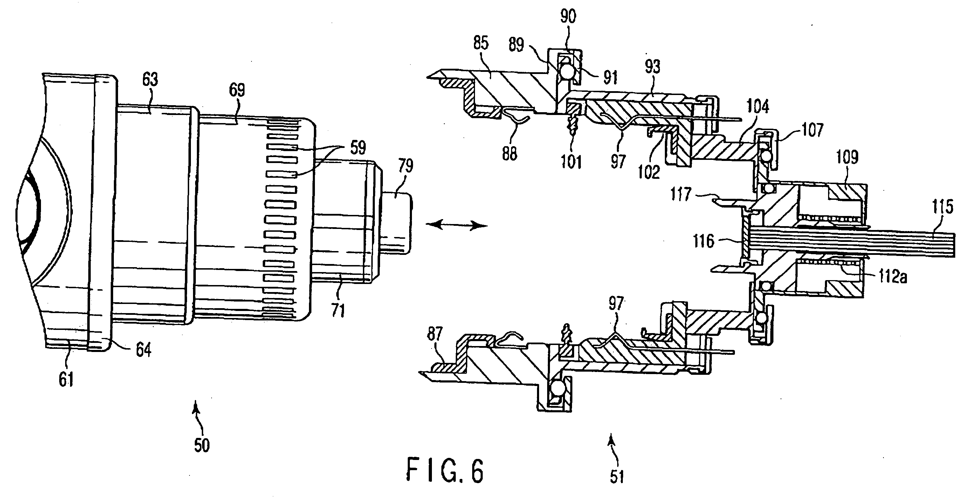

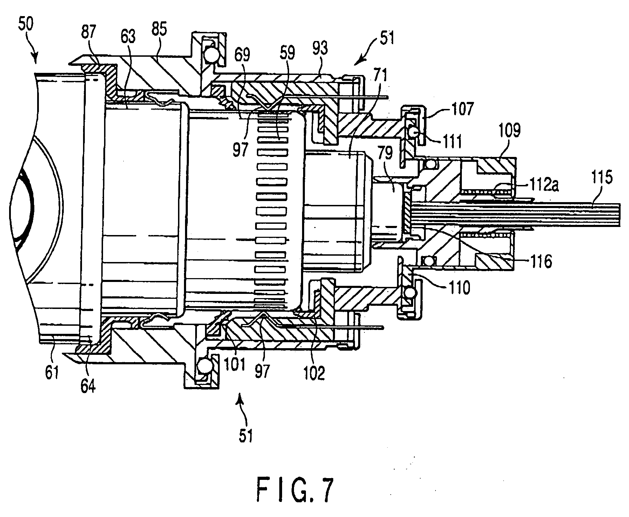

FIG. 4 to FIG. 7 show the internal structure of a coupling section between the insertion-section-side body 50 and the power unit 51. FIG. 4 is a longitudinal cross-sectional view showing a non-connected state (pre-coupling state) of the coupling section between the insertion-section-side body 50 and the power unit 51, and FIG. 5 is a longitudinal cross-sectional side view showing a connected state of the coupling section. FIG. 6 is a side view showing a non-connected state of the insertion-section-side body 50 and power unit 51, with the power unit 51 alone being shown in cross section, and FIG. 7 is a side view showing a connected state of the insertion-section-side body 50 and power unit 51, with the power unit 51 alone being shown in cross section.

To begin with, the structure of the coupling section on the insertion-section-side body 50 side is described. A chassis 61, which constitutes a body portion of the angular cylindrical portion 55, is provided in the insertion-section-side body 50. The chassis 61 is formed of a metallic material with rigidity, such as aluminum, or resin material. A rear end portion of the chassis 61 is opened. An engaging stepped portion 62 is formed at an end edge portion surrounding the rear-end opening of the chassis 61.

A cylindrical engaging member 63 is provided on the rear end portion of the chassis 61. The engaging member 63 constitutes a part of the circular cylindrical portion 56. The engaging member 63 is formed of a metallic material with rigidity, such as aluminum. A closing portion 65 for closing the rear-end opening of the chassis 61 is provided at a proximal end portion of the engaging member 63. The closing portion 65 has a flange portion 64 at an outer peripheral part thereof. A small-diameter portion 66, which is opened, is provided at a distal end portion of the engaging member 63.

The flange portion 64 of the engaging member 63 is provided with an annular groove 67 which is engaged with the engaging stepped portion 62 of the chassis 61. The engaging stepped portion 62 and the annular groove 67 are watertightly sealed by an O ring 68.

The engaging member 63 is provided with a circular cylindrical insertion-side connector body 69. The insertion-side connector body 69 constitutes a part of the circular cylindrical portion 56. The insertion-side connector body 69 is formed of an electrically insulating material.

A peripheral edge portion of the opening of the proximal end portion of the insertion-side connector body 69 is watertightly engaged with the small-diameter portion 66 of the engaging member 63 via an O ring 70. An annular thick partition wall 69a is provided on a peripheral edge of the distal-end opening portion of the insertion-side connector body 69. A projecting cylindrical portion 71 with a small diameter is integrally formed on a distal end portion of the partition wall 69a. The axis of the projecting cylindrical portion 71 is coaxial with the axis of the insertion-side connector body 69.

The electrical contact portions 59 are disposed on the outer peripheral surface of the insertion-side connector body 69. A lead portion 59a of each electrical contact portion 59 penetrates the partition wall 69a of the insertion-side connector body 69 and extends into the inside of the insertion-side connector body 69.

A gasket 72 of an elastic material is fixed in a pressure-contact state in the insertion-side connector body 69 by a gasket pressing member 73. The lead portions 59a of the electrical contact portions 59 penetrate the gasket 72 and are kept watertight. Further, a flexible connector 74, which is connected to the lead portions 59a, is fixed to the gasket pressing member 73. A connector 75 is connected to the flexible connector 74.

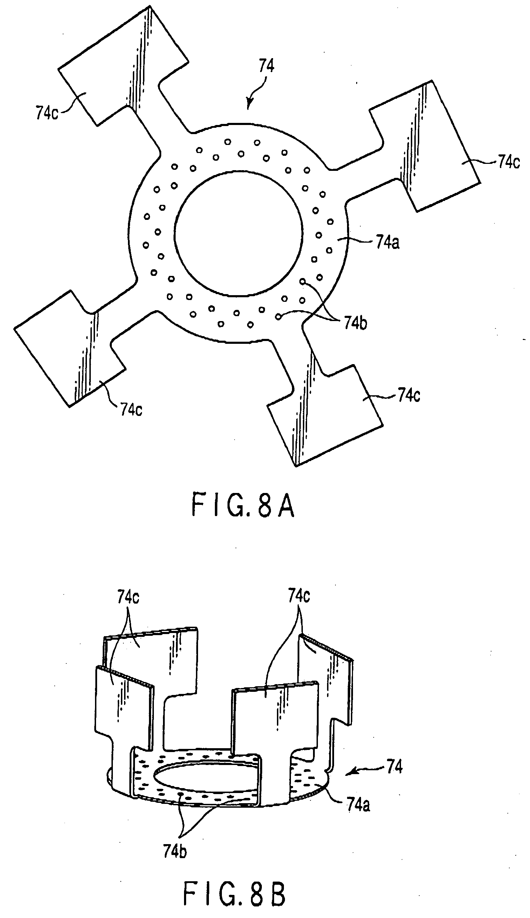

FIG. 8A shows the flexible connector 74. The flexible connector 74 includes an annular base portion 74a and a plurality of tongue-shaped terminal pieces 74c. A great number of connection holes 74b, which are connected to the lead portions 59a, are provided in the annular base portion 74a. Further, the plural terminal pieces 74c are integrally provided on an outer peripheral portion of the annular base portion 74a in a radially projecting state. As shown in FIG. 8B, the terminal pieces 74c are bent and erected in the same direction. The connector 75 is disposed on each terminal piece 74c.

As shown in FIG. 4, a cylindrical portion 76 is integrally formed at a central part of the closing portion 65 of the engaging member 63. The cylindrical portion 76 is projected in a direction toward the rear end portion of the circular cylindrical portion 56. A female screw portion 76a is formed on an inner peripheral surface of the cylindrical portion 76. A proximal end portion of a light guide fixing member 77, which is formed of a pipe, is fixed by screwing in the female screw portion 76a.

A distal end portion of the light guide fixing member 77 projects into the inside of the projecting cylindrical portion 71 of the insertion-side connector body 69. The light guide 46 is inserted in the light guide fixing member 77.

A pipe-shaped light guide engaging member 78 is engaged and fixed on a distal end portion of the light guide 46. A proximal end portion of the light guide engaging member 78 is engaged with an inner peripheral surface of the light guide fixing member 77. A lens frame 79 is fixed to a distal end portion of the light guide engaging member 78. An optical lens 80 is mounted in the lens frame 79. A cylindrical electrical connector fixing member 81 is engaged with an outer peripheral surface of the lens frame 79. An O ring 82 is provided between the outer peripheral surface of the lens frame 79 and the inner peripheral surface of the electrical connector fixing member 81, and watertight sealing is effected therebetween.

An O ring 83 is also provided between the outer peripheral surface of the electrical connector fixing member 81 and the inner peripheral surface of the projecting cylindrical portion 71 of the insertion-side connector body 69. Watertight sealing is effected by the O ring 83 between the electrical connector fixing member 81 and the projecting cylindrical portion 71 of the insertion-side connector body 69.

A flange portion 81a is provided at a distal end portion of the electrical connector fixing member 81. An electrical connector protection member 84, which is formed of a metal, is provided between the flange portion 81a and the projecting cylindrical portion 71. Accordingly, watertight sealing is effected by the O ring 83 between the insertion-side connector body 69, which is provided with the electrical contact portions 59, and the electrical connector fixing member 81, watertight sealing is effected by the O ring 82 between the electrical connector fixing member 81 and the lens frame 79, and watertight sealing is effected by the O ring 70 between the insertion-side connector body 69 and the engaging member 63.

Next, the structure of the coupling section on the power unit 51 side is described. A body portion of the power unit 51 is provided with a chassis 85 which serves as a stationary base plate. The chassis 85 is formed of a metallic material with rigidity, such as aluminum. The chassis 85 has a cavity portion 86. The engaging member 63 of the insertion-section-side body 50 is inserted into the cavity portion 86 of the chassis 85.

A frame-shaped engagement reception portion 87 is provided on an inner peripheral surface of the cavity portion 86. A plurality of fingers 88, which are formed of metallic resilient members such as plate springs, are provided in a circumferential direction on the engagement reception portion 87.

A flange receiving portion 89, which constitutes a first standard surface, is provided at a distal end portion of the chassis 85. In the flange receiving portion 89, an annular groove 92 is formed by a peripheral wall portion 90 and a hold member 91. The hold member 91 is fixed on the peripheral wall portion 90.

A circular cylindrical casing 93 is provided as a first connector portion at a distal end side of the chassis 85. A flange portion 94 is provided at a proximal end portion of the casing 93. The flange portion 94 is engaged with the annular groove 92 of the chassis 85. The inside diameter of the annular groove 92 is set to be greater than the outside diameter of the flange portion 94 of the casing 93. Thus, the casing 93 is radially movable relative to the annular groove 92. Watertight sealing is effected by an O ring 95 between the flange portion 94 and the hold member 91. Accordingly, such support means is constituted that the flange portion 94 is three-dimensionally oscillatably supported on the flange receiving portion 89.

A circular cylindrical terminal fixing member 96 is provided in the casing 93. A plurality of electrical terminal portions 97 are provided on the inner peripheral surface of the terminal fixing member 96. The plural electrical terminal portions 97 are disposed at positions corresponding to the electrical contact portions 59 of the insertion-side connector body 69. The electrical terminal portions 97 project inward from the inner peripheral surface of the terminal fixing member 96.

A gasket 98 of an elastic material is fixed on the end face of the terminal fixing member 96 in a pressure-contact state by a gasket pressing member 99. Further, the gasket pressing member 99 is held and fixed by a terminal fixing ring 100 which is fixed to the casing 93 by screwing. A lead portion 97a of each electrical terminal portion 97 penetrates the gasket 98 and is kept watertight.

The terminal fixing member 96 is provided with two wipers (first wiper 101 and second wiper 102).

The first wiper 101 is formed of an annular rubber member and is disposed along the inner peripheral surface of the casing 93. The first wiper 101 includes a waist portion 101a at a proximal portion thereof, as shown in cross section, and an arcuate sliding contact portion 101b at a distal end portion thereof. The first wiper 101 is formed such that the inside diameter of the sliding contact portion 101b is slightly less than the outside diameter of the insertion-side connector body 69. Thereby, the first wiper 101 is bent in a right-and-left direction, with the waist portion 101a functioning as a fulcrum, in accordance with insertion/removal of the insertion-side connector body 69 in/from the terminal fixing member 96 of the power unit 51. In this state, the sliding contact portion 101b of the first wiper 101 comes in sliding contact with the outer peripheral surface of the insertion-side connector body 69. As a result, the first wiper 101 functions as removing means for removing moisture, which is disposed adjacent to the electrical terminal portion 97 on the proximal end side of the electrical terminal portion 97.

The second wiper 102 is also formed of an annular rubber member, and is disposed along the inner peripheral surface of the terminal fixing member 96. The second wiper 102, as shown in cross section, includes a proximal portion 102a and a sliding contact portion 102b. The proximal portion 102a of the second wiper 102 is fixed to the terminal fixing member 96 by a wiper fixing ring 103. The sliding contact portion 102b is formed in a bent shape in such a state as to project inward from an outer end portion of the proximal portion 102a. The second wiper 102 is formed such that the inside diameter of the sliding contact portion 102b is slightly less than the outside diameter of the insertion-side connector body 69. In addition, the second wiper 102 is formed such that the sliding contact portion 102b comes in sliding contact with the outer peripheral surface of the insertion-side connector body 69 in accordance with insertion/removal of the insertion-side connector body 69 in/from the terminal fixing member 96 of the power unit 51. As a result, the second wiper 102 functions as prevention means which is disposed adjacent to the electrical terminal portion 97 on the distal end side of the terminal fixing member 96, and prevents entrance of moisture in a direction different from the direction in which the first wiper 101 removes moisture.

As described above, the terminal fixing member 96 is provided with the first wiper 101 and second wiper 102 before and behind the electrical terminal portion 97. Thereby, in accordance with insertion/removal of the insertion-side connector body 69 in/from the terminal fixing member 96 of the power unit 51, moisture adhering to the surface of the terminal fixing member 96 can be removed by the first wiper 101. Furthermore, the second wiper 102 is configured to prevent moisture, which adheres to the distal end side of the terminal fixing member 96, from flowing toward the electrical contact portion 59 by force of gravity.

A circular cylindrical light guide fixing base plate 104 is fixed to a distal end portion of the terminal fixing member 96. A flange receiving portion 105 is provided at a distal end portion of the light guide fixing base plate 104. The flange receiving portion 105 constitutes a second standard surface. In the flange receiving portion 105, an annular groove 108 is formed by a peripheral wall portion 106 and a hold member 107. The hold member 107 is fixed to the peripheral wall portion 106.

A circular cylindrical light guide fixing member 109 serving as a second connector portion is provided on the distal end side of the light guide fixing base plate 104. A flange portion 110 is provided at a proximal end portion of the light guide fixing member 109. The flange portion 110 is engaged in the annular groove 108 of the light guide fixing base plate 104. The inside diameter of the annular groove 108 is set to be greater than the outside diameter of the flange portion 110 of the light guide fixing member 109. Thus, the light guide fixing member 109 is radially movable relative to the annular groove 108. Watertight sealing is effected by an O ring 111 between the flange portion 110 and the hold member 107. Accordingly, such support means is constituted that the flange portion 110 is three-dimensionally oscillatably supported on the flange receiving portion 105.

A light guide engaging member 112 is received in the light guide fixing member 109. Sealing is effected by an O ring 113 between the outer peripheral surface of the light guide engaging member 112 and the inner peripheral surface of the light guide fixing member 109. The light guide engaging member 112 is axially movable relative to the light guide fixing member 109. Further, a spring 112a is provided between the light guide fixing member 109 and the light guide engaging member 112. The spring 112a resiliently urges the light guide engaging member 112 toward the proximal end side, relative to the light guide fixing member 109.

A through-hole 114 is provided in the axial direction in a central part of the light guide engaging member 112. The light guide 115 is mounted in the through-hole 114. An optical lens 116, which is formed of a flat plate glass is provided on the end face of the light guide 115 in a closely attached state. The light guide engaging member 112 is provided with an engaging cylinder 117. A lens frame 118 is fixed to the engaging cylinder 117. The optical lens 116 is mounted in the lens frame 118.

Next, the operation of the present embodiment having the above-described structure is described. To begin with, a description is given of an operation in which the insertion-side connector body 69 of the insertion-section-side body 50 is inserted in the cavity portion 86 of the power unit 51 and the insertion-section-side body 50 and the power unit 51 are electrically and optically connected.

To begin with, the insertion-section-side body 50 is grasped and the insertion-side connector body 69 is inserted into the cavity portion 86. At this time, the outer peripheral surface of the insertion-side connector body 69 comes in contact with the fingers 88. Accordingly, the insertion-side connector body 69 is resiliently positioned toward the center of the cavity portion 86. If the insertion-side connector body 69 is inserted in the cavity portion 86, the distal end portion of the outer peripheral surface of the insertion-side connector body 69 first comes in contact with the sliding contact portion 101b of the first wiper 101.

Then, if the insertion-side connector body 69 is inserted deeply into the cavity portion 86, sliding movement occurs between the outer peripheral surface of the insertion-side connector body 69 and the sliding contact portion 101b of the first wiper 101. At this time, the first wiper 101 is bent toward the deep part of the cavity portion 86, with the waist portion 101a functioning as a fulcrum, and the sliding contact portion 101b comes in sliding contact with the outer peripheral surface of the insertion-side connector body 69. Accordingly, even if moisture, which adheres at the time of cleaning, remains on the outer peripheral surface of the insertion-side connector body 69, the moisture is removed by the first wiper 101. The plural electrical contact portions 59 are provided on the outer peripheral surface of the insertion-side connector body 69. Moisture, which may possibly adhere to the surfaces of the electrical contact portions 59, is also removed by the first wiper 101.

Then, if the insertion-side connector body 69 is inserted more deeply into the cavity portion 86, the electrical contact portions 59 are opposed to the electrical terminal portions 97. Subsequently, the electrical contact portions 59 are put in contact with the electrical terminal portions 97, and the insertion-section-side body 50 and power unit 51 are rendered electrically conductive. If the insertion-side connector body 69 is inserted into the deepest part of the cavity portion 86, the outer peripheral surface of the distal end portion of the insertion-side connector body 69 comes in sliding contact with the sliding contact portion 102b of the second wiper 102. Thereby, the sliding contact portion 102b is bent and brought into close contact with the outer peripheral surface of the distal end portion of the insertion-side connector body 69. Specifically, the outer peripheral surface of the distal end portion of the insertion-side connector body 69 is shut off from the projecting cylindrical portion 71 by the second wiper 102. Thus, even if moisture adheres between the distal end side of the insertion-side connector body 69 and the projecting cylindrical portion 71, the second wiper 102 prevents the moisture from flowing toward the electrical contact portion 59. Specifically, in the state of use of the endoscope 20, it is possible that the insertion-section-side body 50 is positioned on the lower side in a vertical position and moisture between the distal end side of the insertion-side connector body 69 and the projecting cylindrical portion 71 flows, by force of gravity, toward the electrical contact portions 59 of the insertion-section-side body 50. In the present embodiment, since the second wiper 102 is provided, the moisture, which tries to flow toward the electrical contact portions 59, can be shut out.

If the insertion-side connector body 69 is inserted into the deepest part of the cavity portion 86 in this manner, the lens frame 79 of the insertion-side connector body 69 is engaged in the engaging cylinder 117 of the light guide engaging member 112. Thereby, the optical lens 80 and optical lens 116 are opposed, and the power unit 51 and insertion-section-side body 50 are also optically connected.

The flange portion 94 of the casing 93 is radially movable relative to the annular groove 92 of the chassis 85 on the power unit 51 side. The flange portion 94 of the casing 93 is resiliently pressed on the chassis 85 by the O ring 95. Thus, the casing 93 is oscillatably supported, and the terminal fixing member 96 is three-dimensionally oscillatable. Moreover, the flange portion 110 of the light guide fixing member 109 is radially movable relative to the annular groove 108 of the light guide fixing base plate 104. The light guide fixing member 109 is resiliently pressed on the light guide fixing base plate 104 by the O ring 111, and the light guide fixing member 109 is three-dimensionally oscillatable.

Accordingly, when the insertion-side connector body 69 is inserted in the cavity portion 86, even if the insertion distal-end side of the insertion-side connector body 69 oscillates in the radial direction, the terminal fixing member 96 and light guide fixing member 109 three-dimensionally oscillate and follow in a manner to follow the oscillation of the insertion-side connector body 69. Therefore, when the insertion-section-side body 50 is grasped and the insertion-side connector body 69 is inserted into the cavity portion 86, even if the insertion distal-end side of the insertion-side connector body 69 is misaligned in the radial direction, the insertion-side connector body 69 can smoothly be inserted without a sensation of the insertion distal-end side of the insertion-side connector body 69 being caught. The electrical contact portions 59 are put in contact with, and electrically connected to, the electric terminal portions 97, and the lens frame 79 is engaged in the engaging cylinder 117 of the light guide engaging member 112 and optical connection is ensured.

Furthermore, the light guide engaging member 112 is engaged with the light guide fixing member 109, and the light guide engaging member 112 is resiliently pressed on the flange receiving portion 105 of the light guide fixing base plate 104 by the spring 112a. Therefore, an impact force in the direction of insertion, which occurs when the lens frame 79 of the insertion-side connector body 69 is engaged in the engaging cylinder 117 of the light guide engaging member 112, can be absorbed by the spring 112a, and at the same time the optical lenses 80 and 116 can be put in close contact.

The following advantageous effects can be obtained by the above-described structure. Specifically, in the present embodiment, the terminal fixing member 96 of the power unit 51 is provided with the first wiper 101 and second wiper 102 before and behind the electrical terminal portion 97. Thereby, in accordance with insertion/removal of the insertion-side connector body 69 in/from the terminal fixing member 96 of the power unit 51, moisture adhering to the surface of the terminal fixing member 96 can be removed by the first wiper 101. Furthermore, the second wiper 102 can prevent moisture, which adheres to the distal end side of the terminal fixing member 96, from flowing toward the electrical contact portion 59 by force of gravity. Therefore, even if moisture adheres to the electrical contact portion 59 when the power unit 51 and the insertion-section-side body 50 are connected, the moisture on the electrical contact portion 59 can be removed in the process of connecting the power unit 51 and the insertion-section-side body 50. Moreover, there is no concern that moisture that is removed or moisture that is not removed and remains on the connector section adheres to the electrical contact portion 59, and the reliability can be improved.

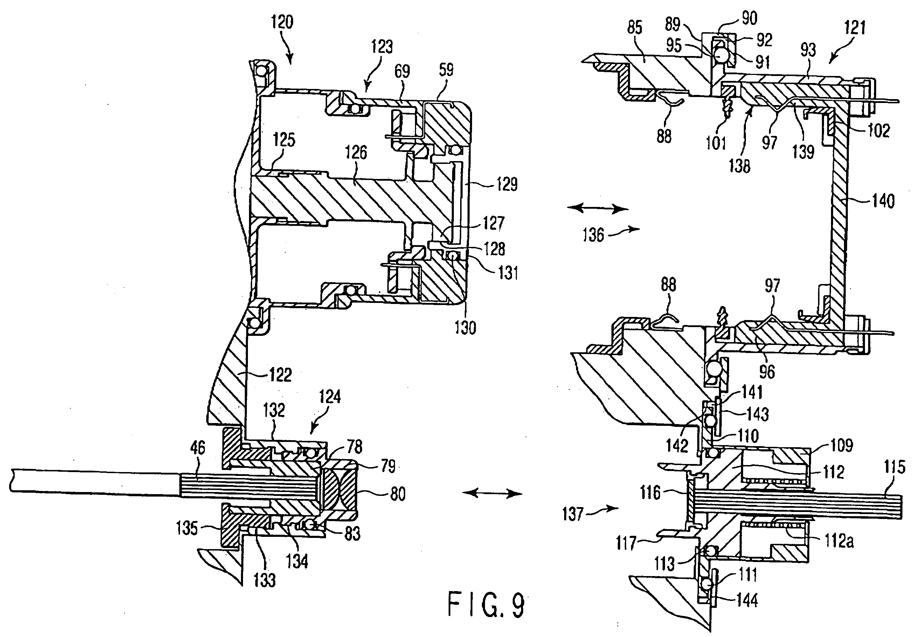

FIG. 9 shows a second embodiment of the present invention. The structural parts common to those in the first embodiment are denoted by like reference numerals, and a description thereof is omitted here. FIG. 9 is a longitudinal cross-sectional view showing the internal structure in a non-connected state of the coupling section between an insertion-section-side body 120 and a power unit 121 according to the present embodiment.

To begin with, the insertion-section-side body 120 side is described. A chassis 122, which is formed of a metallic material with rigidity, such as aluminum, is provided in the insertion-section-side body 120. An electrical connector member 123 and an optical connector member 124 are adjacently provided in parallel at a rear end portion of the chassis 122. Like the first embodiment, the electrical connector member 123 is provided with an insertion-side connector body 69. Electrical contact portions 59 are disposed on the insertion-side connector body 69.

A cylindrical portion 125, which projects toward the rear end portion, is provided on the chassis 122. An electrical connector fixing column 126 is fixed by screwing in the cylindrical portion 125. The electrical connector fixing column 126 extends through a central part of the insertion-side connector body 69, and projects to the distal end side of the insertion-side connector body 69. A flange portion 127 is provided at a distal end portion of the electrical connector fixing column 126. A screw portion 128 is formed on the outer peripheral surface of the flange portion 127. A cap 129 is fixed by screwing on the screw portion 128. The outer peripheral surface of the cap 129 is put in close contact with an engaging hole 131 of the insertion-side connector body 69 via an O ring 130. Thereby, the insertion-side connector body 69 is supported by the electrical connector fixing column 126, and at the same time watertight sealing is effected.

The optical connector member 124 includes a circular cylindrical portion 132 which projects rearward. Screw portions 133 and 134 are provided on a proximal end portion and a distal end portion of the inner peripheral surface of the circular cylindrical portion 132. A light guide fixing frame 135 is fixed by screwing, from the inside of the chassis 122, on the screw portion 133 of the proximal end portion. Thereby, the optical connector member 124 is reinforced. Like the first embodiment, a light guide engaging member 78 for engagement with the light guide 46 is inserted in the light guide fixing frame 135. A lens frame 79, in which an optical lens 80 is mounted, is engaged with the light guide engaging member 78. The lens frame 79 is fixed by screwing on the screw portion 134 of the optical connector member 124. Further, watertight sealing is effected by an O ring 83 between the outer peripheral surface of the lens frame 79 and the optical connector member 124.

Next, the power unit 121 side is described. A chassis 85, which constitutes a body portion of the power unit 121, is provided with an electrical connector cavity portion 136 and an optical connector cavity portion 137, which are adjacent to each other in parallel. The electrical connector cavity portion 136 and optical connector cavity portion 137 correspond to the electrical connector member 123 and optical connector member 124 on the insertion-section-side body 120 side, respectively.

In the electrical connector cavity portion 136, a circular cylindrical casing 93 is provided on the distal end side of the chassis 85. A terminal fixing member 138 is provided inside the casing 93. The terminal fixing member 138 is provided with a circular cylindrical portion 139 and a closing portion 140 which closes a distal-end opening of the circular cylindrical portion 139.

Like the first embodiment, a plurality of electrical terminal portions 97 are disposed on the inner peripheral surface of the circular cylindrical portion 139. The respective electrical terminal portions 97 are disposed at positions corresponding to the electrical contact portions 59 on the insertion-section-side body 120 side. A first wiper 101 is provided on a proximal end side of the circular cylindrical portion 139. The first wiper 101 is disposed adjacent to the electrical terminal portions 97 and functions as removing means for removing moisture. A second wiper 102 is provided on a distal end side of the circular cylindrical portion 139. The second wiper 102 is disposed on the inner surface of the closing portion 140, and functions as prevention means for preventing entrance of moisture in a direction different from the direction in which the first wiper 101 removes moisture.

In the optical connector cavity portion 137, a circular cylindrical light guide fixing member 109 is provided. The light guide fixing member 109 is fixed on a distal end side of the chassis 85. A flange receiving portion 141, which constitutes a second standard surface, is provided at a front end portion of the chassis 85. An annular groove 144 is formed in the flange receiving portion 141. The annular groove 144 is formed by a peripheral wall portion 142 and a hold member 143 of the flange receiving portion 141. The hold member 143 is fixed to the peripheral wall portion 142 of the flange receiving portion 141.

A flange portion 110 is provided at a proximal end portion of the light guide fixing member 109. The flange portion 110 is engaged in the annular groove 144. The inside diameter of the annular groove 144 is set to be greater than the outside diameter of the flange portion 110 of the light guide fixing member 109. Thereby, the light guide fixing member 109 is radially movable relative to the annular groove 144. Watertight sealing is effected by an O ring 111 between the flange portion 110 and the hold member 143. Accordingly, the flange receiving portion 89 serving as the first standard surface and the flange receiving portion 141 serving as the second standard surface are positioned substantially in the same plane. Thus, such support means is constituted that the flange portion 110 is three-dimensionally oscillatably supported on the flange receiving portion 141 serving as the second standard surface.

Next, a description is given of the operation of electrically and optically connecting the insertion-section-side body 120 and the power unit 121. To begin with, the insertion-section-side body 120 is grasped and the electrical connector member 123 and optical connector member 124 are aligned with, and inserted in, the electrical connector cavity portion 136 and optical connector cavity portion 137 of the power unit 121.

At this time, a distal end portion of the outer peripheral surface of the insertion-side connector body 69 of the electrical connector member 123 comes in contact with the sliding contact portion 101b of the first wiper 101. Then, if the insertion-side connector body 69 is inserted deeply into the electrical connector cavity portion 136, the sliding contact portion 101b of the first wiper 101 slidingly moves over the outer peripheral surface of the insertion-side connector body 69. At this time, the first wiper 101 is bent toward the deep part of the electrical connector cavity portion 136, with the waist portion 101a functioning as a fulcrum. Thus, the sliding contact portion 101b comes in sliding contact with the outer peripheral surface of the insertion-side connector body 69. Accordingly, like the first embodiment, even if moisture, which adheres at the time of cleaning, remains on the outer peripheral surface of the insertion-side connector body 69, the moisture is removed by the first wiper 101. At this time, moisture on the surfaces of the electrical contact portions 59 is also removed by the first wiper 101.

Then, if the insertion-side connector body 69 is inserted more deeply into the electrical connector cavity portion 136, the electrical contact portions 59 are opposed to the electrical terminal portions 97, and the electrical contact portions 59 are put in contact with the electrical terminal portions 97. Thereby, the insertion-section-side body 120 and power unit 121 are rendered electrically conductive. In addition, the outer peripheral surface of the distal end portion of the insertion-side connector body 69 comes in sliding contact with the sliding contact portion 102b of the second wiper 102. At this time, the sliding contact portion 102b is bent and brought into close contact with the outer peripheral surface of the distal end portion of the insertion-side connector body 69. Thus, even if moisture adheres to the distal end side of the insertion-side connector body 69, the second wiper 102 prevents the moisture from flowing toward the electrical contact portion 59.

If the insertion-side connector body 69 is inserted into the cavity portion 136 in this manner, the lens frame 79 of the insertion-side connector body 69 is engaged in the engaging cylinder 117 of the light guide engaging member 112. Thereby, the optical lens 80 and optical lens 116 are opposed, and the power unit 121 and insertion-section-side body 120 are also optically connected.

The flange portion 94 of the casing 93 is radially movable relative to the annular groove 92 of the chassis 85 on the power unit 121 side. At this time, the flange portion 94 of the casing 93 is resiliently pressed on the chassis 85 by the O ring 95. Thus, the casing 93 is oscillatably supported, and the terminal fixing member 138 is three-dimensionally oscillatable. Moreover, the flange portion 110 of the light guide fixing member 109 is radially movable relative to the annular groove 144 of the chassis 85. At this time, the light guide fixing member 109 is resiliently pressed on the chassis 85 by the O ring 111, and the light guide fixing member 109 is three-dimensionally oscillatable.

Accordingly, like the first embodiment, when the insertion-side connector body 69 is inserted in the electrical connector cavity portion 136, even if the insertion distal-end side of the insertion-side connector body 69 oscillates in the radial direction, the terminal fixing member 138 three-dimensionally oscillates in a manner to follow the oscillation of the insertion-side connector body 69. In addition, even if the insertion distal-end side of the optical connector member 124 oscillates in the radial direction, the light guide fixing member 109 three-dimensionally oscillates in a manner to follow the oscillation of the optical connector member 124. Therefore, when the insertion-section-side body 120 is grasped and coupled to the power unit 121, even if the insertion-section-side body 120 and the power unit 121 are misaligned in the radial direction, the insertion-section-side body 120 can smoothly be inserted without a sensation of the insertion-section-side body 120 being caught. The electrical contact portions 59 are put in contact with, and electrically connected to, the electric terminal portions 97, and at the same time the lens frame 79 is engaged in the engaging cylinder 117 of the light guide engaging member 112 and optical connection is ensured.

In the present embodiment, too, the terminal fixing member 138 of the power unit 121 is provided with the first wiper 101 and second wiper 102 before and behind the electrical terminal portion 97. Thereby, in accordance with insertion/removal of the insertion-side connector body 69 in/from the terminal fixing member 138 of the power unit 121, moisture adhering to the surface of the terminal fixing member 138 can be removed by the first wiper 101. Furthermore, the second wiper 102 can prevent moisture, which adheres to the distal end side of the terminal fixing member 138, from flowing toward the electrical contact portion 59 by force of gravity. Therefore, even if moisture adheres to the electrical contact portion 59 when the power unit 121 and the insertion-section-side body 120 are connected, the moisture on the electrical contact portion 59 can be removed in the process of connecting the power unit 121 and the insertion-section-side body 120. Moreover, there is no concern that moisture that is removed or moisture that is not removed and remains on the connector section adheres to the electrical contact portion 59, and the reliability can be improved.

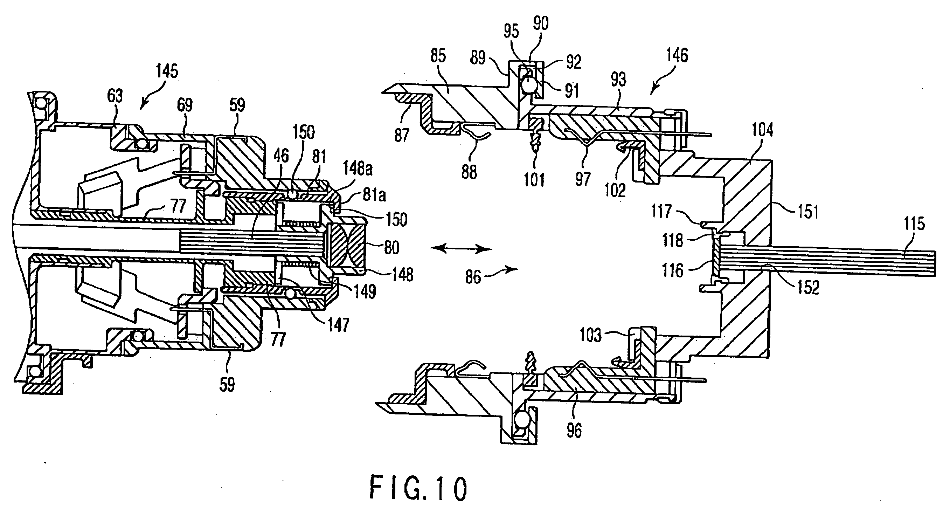

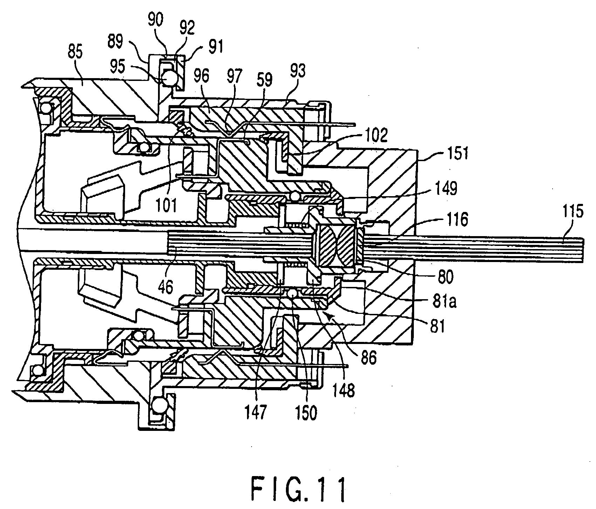

FIG. 10 and FIG. 11 show a third embodiment of the present invention. The structural parts common to those in the first embodiment are denoted by like reference numerals, and a description thereof is omitted. FIG. 10 is a longitudinal cross-sectional view showing a non-connected state of the coupling section between an insertion-section-side body 145 and a power unit 146 according to the present embodiment, and FIG. 11 is a longitudinal cross-sectional view showing a connected state of the insertion-section-side body 145 and power unit 146.

A spring hold member 147 is provided at a distal end portion of the light guide fixing member 77 of the insertion-section-side body 145. A lens frame 148 is engaged with and fixed to the light guide 46. An optical lens 80 is mounted in the lens frame 148. A spring 149 is provided, in a compressed state, between the back surface of the lens frame 148 and the spring hold member 147. Further, a flange portion 148a is provided on the lens frame 148. The flange portion 148a is engaged with the inner peripheral surface of an electrical connector fixing member 81 via the space. The flange portion 148a is opposed to a flange portion 81a of the electrical connector fixing member 81. Watertight sealing is effected by an O ring 150 between the flange portions 148a and 81a. Thus, the lens frame 148 is radially movable relative to the electrical connector fixing member 81, and is supported by the spring 49 and is axially movable.

Next, the power unit 146 is described. A circular cylindrical light guide fixing base plate 104 is fixed to a distal end portion of the terminal fixing member 96 that is provided inside the casing 93. A closing portion 151 is provided at a distal end portion of the light guide fixing base plate 104. A through-hole 152 is provided in the axial direction in a central part of the closing portion 151. The light guide 115 is mounted in the through-hole 152. An optical lens 116, which is formed of a flat plate glass, is provided on the end face of the light guide 115 in a closely attached state. The optical lens 116 is mounted in the lens frame 118. The lens frame 118 is fixed to an engaging cylinder 117. The engaging cylinder 117 is provided on the light guide fixing base plate 104.

Accordingly, the light guide 115 is fixed to the light guide fixing base plate 104. At this time, the lens frame 148 is resiliently pressed by the spring 149. Thus, an impact force in the direction of insertion, which occurs when the lens frame 148 of the insertion-side connector body 145 is engaged in the engaging cylinder 117 of the light guide fixing base plate 104, can be absorbed by the spring 149, and at the same time the optical lense 80 and the optical lense 116 can be put in close contact.

According to the present embodiment, like the first embodiment, the terminal fixing member 96 of the power unit 146 is provided with the first wiper 101 and second wiper 102 before and behind the electrical terminal portion 97. Thereby, in accordance with insertion/removal of the insertion-side connector body 69 in/from the terminal fixing member 96 of the power unit 146, moisture adhering to the surface of the terminal fixing member 96 can be removed by the first wiper 101. Furthermore, the second wiper 102 can prevent moisture, which adheres to the distal end side of the terminal fixing member 96, from flowing toward the electrical contact portion 59 by force of gravity. Therefore, even if moisture adheres to the electrical contact portion 59 when the power unit 146 and the insertion-section-side body 145 are connected, the moisture on the electrical contact portion 59 can be removed in the process of connecting the power unit 146 and the insertion-section-side body 145. Moreover, there is no concern that moisture that is removed or moisture that is not removed and remains on the connector section adheres to the electrical contact portion 59, and the reliability can be improved.

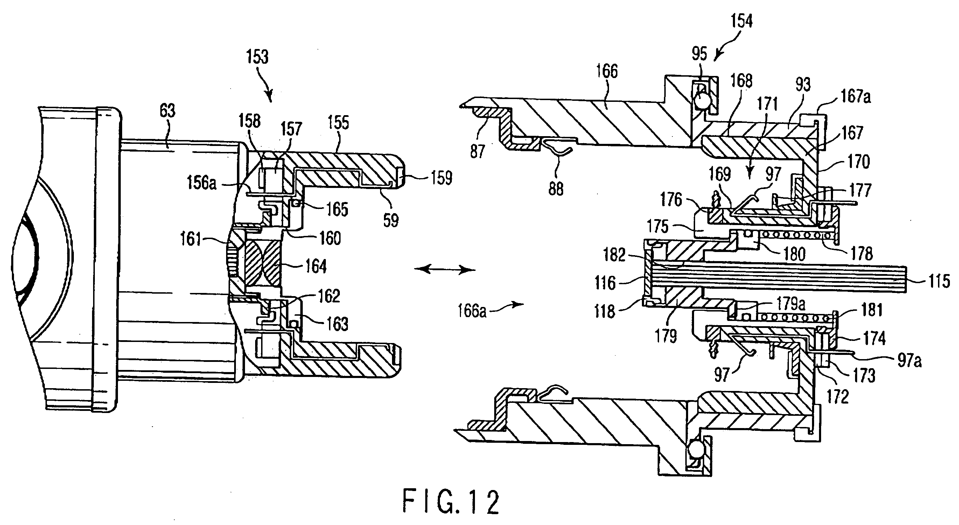

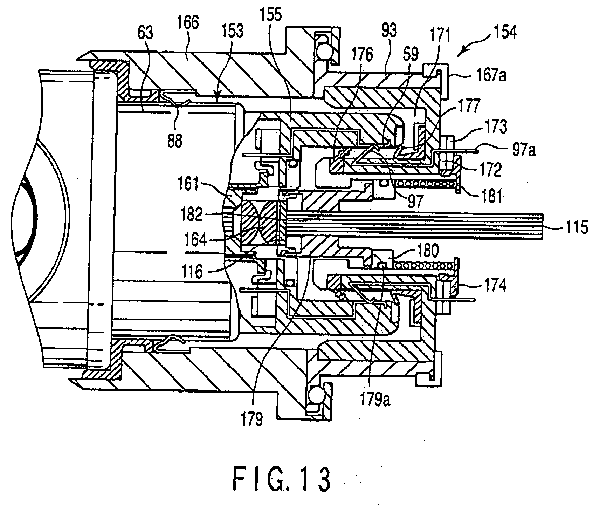

FIG. 12 and FIG. 13 show a fourth embodiment of the invention. The structural parts common to those in the first embodiment are denoted by like reference numerals, and a description thereof is omitted.

FIG. 12 is a longitudinal cross-sectional view showing a non-connected state of the coupling section between an insertion-section-side body 153 and a power unit 154 according to the present embodiment, and FIG. 13 is a longitudinal cross-sectional view showing a connected state of the insertion-section-side body 153 and power unit 154.

To begin with, the insertion-section-side body 153 side is described. The engaging member 63 of the insertion-section-side body 153 is provided with a circular cylindrical insertion-side connector body 155. The insertion-side connector body 155 is formed of an electrically insulating material. A plurality of electrical contact portions 59 are disposed on the inner peripheral surface of the insertion-side connector body 155. A lead portion 59a of each electrical contact portion 59 penetrates the partition wall of the insertion-side connector body 155 and extends into the inside of the insertion-side connector body 155.

A gasket 157 of an elastic material is fixed in a pressure-contact state in the insertion-side connector body 155 by a gasket pressing member 158. The lead portions 59a of the electrical contact portions 59 penetrate the gasket 157 and are kept watertight. Further, an electrical connector protection member 159 is provided at the distal end portion of the insertion-side connector body 155.

A through-hole 160 is provided in the axial direction in a central part of the proximal end portion of the insertion-side connector body 155. In the through-hole 160, a pipe-shaped light guide engaging member 161 is provided in an opposed fashion. A light guide fixing member 162 is engaged with a distal end portion of the light guide engaging member 161. In the through-hole 160, a lens frame 163, which is fixed to the light guide fixing member 162, is provided. An optical lens 164 is mounted in the lens frame 163. An O ring 165 is fitted on the outer peripheral surface of the lens frame 163, and watertight sealing is effected between the outer peripheral surface of the lens frame 163 and the through-hole 160.

Next, the power unit 154 side is described. A body portion of the power unit 154 is provided with a chassis 166 as a stationary base plate. The chassis 166 is formed of a metallic material with rigidity, such as aluminum. The chassis 166 is provided with a circular cylindrical cavity portion 166a. The insertion-section-side connector body 155 is inserted into the cavity portion 166a. An engagement reception portion 87 is provided on the inner peripheral surface of the cavity portion 166a. A plurality of fingers 88, which are formed of metallic resilient members, are provided in a circumferential direction on the engagement reception portion 87.

A circular cylindrical casing 93 is provided at a distal end portion of the chassis 166. A terminal fixing member 167 is fixed inside the casing 93 by a fixing ring 167a. The terminal fixing member 167 includes an outer peripheral wall portion 168, an inner peripheral wall portion 169 and a closing wall portion 170. An annular engaging portion 171 is provided between the outer peripheral wall portion 168, inner peripheral wall portion 169 and closing wall portion 170. The insertion-side connector body 155 is inserted in the engaging portion 171.

A plurality of electrical terminal portions 97 are disposed on an outer peripheral surface of the inner peripheral wall portion 169 of the terminal fixing member 167. The respective electrical terminal portions 97 are disposed at positions corresponding to the electrical contact portions 59 of the insertion-side connector body 155. A gasket 172 of an elastic material is fixed in a pressure-contact state on the closing wall portion 170 of the terminal fixing member 167 by a gasket pressing member 173. Lead portions 97a of the electrical terminal portions 97 penetrate the gasket 172 and are kept watertight. In addition, a gasket hold member 174 is provided on the gasket pressing member 173. The gasket hold member 174 is fixed by screwing to an electrical connector protection member 175 which is fixed to the terminal fixing member 167.

A first wiper 176 is provided on an outer peripheral surface of the inner peripheral wall portion 169 of the terminal fixing member 167. The first wiper 176 functions as removing means for removing moisture, which is disposed adjacent to the electrical terminal portion 97. A second wiper 177 is provided on a closing wall portion 170 side of the inner peripheral wall portion 169 of the terminal fixing member 167. The second wiper 177 functions as prevention means which is disposed adjacent to the electrical terminal portion 97 and prevents entrance of moisture in a direction different from the direction in which the first wiper 176 removes moisture. The first and second wipers 176 and 177 are formed of annular rubber members along the outer peripheral surface of the inner peripheral wall portion 169. The outside diameter of each of the first and second wipers 176 and 177 is set to be slightly greater than the inside diameter of the insertion-side connector body 155. Thereby, when the insertion-side connector body 155 is inserted/removed in/from the terminal fixing member 167 of the power unit 154, the first and second wipers 176 and 177 come in sliding contact with the inner peripheral surface of the insertion-side connector body 155.

In this manner, the terminal fixing member 167 is provided with the first wiper 176 and second wiper 177 on both sides of the electrical terminal portions 97. When the insertion-side connector body 155 is inserted/removed in/from the terminal fixing member 167 of the power unit 154, the first wiper 176 can remove moisture adhering to the surface of the insertion-side connector body 155. Further, the second wiper 177 is configured to prevent moisture, which adheres to the distal end side of the insertion-side connector body 155, from flowing toward the electrical contact portion 59 by force of gravity.

An annular recess portion 178 is provided in the electrical connector protection member 175. The annular recess portion 178 is formed by a flange receiving portion which constitutes a first standard surface. A flange portion 179a of a light guide engaging member 179 is engaged in the annular recess portion 178. The outside diameter of the flange portion 179a is slightly less than the inside diameter of the annular recess portion 178, and a gap is provided on the outer peripheral surface of the flange portion 179a. A spring hold member 180 is coupled to the flange portion 179a. A spring 181 is provided, in a compressed state, between the spring hold member 180 and the gasket hold member 174. A through-hole 182 is provided in the axial direction in a central part of the light guide engaging member 179. A light guide 115 is mounted in the through-hole 182. An optical lens 116, which is formed of a flat plate glass, is provided, in a closely attached state, to the end face of the light guide 115. The optical lens 116 is mounted in a lens frame 118. Accordingly, the light guide engaging member 179 is radially and axially movable relative to the electrical connector protection member 175, and the spring hold member 180 is resiliently supported by the spring 181.

Next, a description is given of an operation in which the insertion-section-side body 153 and the power unit 154 are electrically and optically connected. To begin with, the insertion-section-side body 153 is grasped and the insertion-side connector body 155 is inserted into the engaging portion 171 of the power unit 154. At this time, the outer peripheral surface of the insertion-side connector body 155 comes in contact with the fingers 88. Accordingly, the insertion-side connector body 155 is resiliently positioned toward the center of the engaging portion 171. Subsequently, if the insertion-side connector body 155 is inserted in the engaging portion 171, the distal end portion of the inner peripheral surface of the insertion-side connector body 155 first comes in contact with the first wiper 176.

Then, the insertion-side connector body 155 is inserted deeply into the engaging portion 171. Thereby, sliding movement occurs between the inner peripheral surface of the insertion-side connector body 155 and the first wiper 176. Accordingly, even if moisture, which adheres at the time of cleaning, remains on the inner peripheral surface of the insertion-side connector body 155, the moisture is removed by the first wiper 176. The plural electrical contact portions 59 are provided on the inner peripheral surface of the insertion-side connector body 155. Moisture, which may possibly adhere to the surfaces of the electrical contact portions 59, is also removed by the first wiper 176.

Then, if the insertion-side connector body 155 is inserted more deeply, the electrical contact portions 59 are opposed to the electrical terminal portions 97, and the electrical contact portions 59 are put in contact with the electrical terminal portions 97. Thereby, the insertion-section-side body 153 and power unit 154 are rendered electrically conductive. If the insertion-side connector body 155 is inserted into the deepest part of the engaging portion 171, the inner peripheral surface of the distal end portion of the insertion-side connector body 155 comes in sliding contact with the second wiper 177. In this state, the second wiper 177 is brought into close contact with the inner peripheral surface of the distal end portion of the insertion-side connector body 155. Specifically, the distal end side of the insertion-side connector body 155 is shut off by the second wiper 177. Thus, even if moisture adheres to the distal end side of the insertion-side connector body 155, the second wiper 177 prevents the moisture from flowing toward the electrical contact portion 59. Specifically, in the state of use of the endoscope 20, it is possible that the insertion-section-side body 153 is positioned on the lower side in a vertical position. Consequently, moisture on the distal end side of the insertion-side connector body 155 may flow, by force of gravity, toward the electrical contact portions 59. In the present embodiment, since the second wiper 177 is provided, the moisture, which tries to flow toward the electrical contact portions 59, can be shut out.

If the insertion-side connector body 155 is inserted into the deepest part of the engaging portion 171 in this manner, the optical lens 164 of the insertion-side connector body 155 and optical lens 116 are opposed. Thereby, the power unit 154 and insertion-section-side body 153 are also optically connected.

The flange portion 94 of the casing 93 is radially movable relative to the annular groove 92 of the chassis 166 on the power unit 154 side. The flange portion 94 of the casing 93 is resiliently pressed on the chassis 166 by the O ring 95. Thus, the casing 93 is oscillatably supported, and the terminal fixing member 167 is three-dimensionally oscillatable. Moreover, the flange portion 179a of the light guide engaging member 179 is radially movable relative to the electrical connector protection member 175. Further, since the light guide engaging member 179 is resiliently pressed and supported by the spring 181, the light guide engaging member 179 is three-dimensionally oscillatable.

Accordingly, when the insertion-side connector body 155 is inserted in the engaging portion 171, even if the insertion distal-end side of the insertion-side connector body 155 oscillates in the radial direction, the terminal fixing member 167 and light guide engaging member 179 three-dimensionally oscillate in a manner to follow the oscillation of the insertion-side connector body 155. Therefore, when the insertion-section-side body 153 is grasped and the insertion-side connector body 155 is inserted into the engaging portion 171, even if the insertion distal-end side of the insertion-side connector body 155 is misaligned in the radial direction, the insertion-side connector body 155 can smoothly be inserted without a sensation of the insertion distal-end side of the insertion-side connector body 155 being caught.

Furthermore, the light guide engaging member 179 is resiliently pressed on the flange portion 175a of the electrical connector protection member 175 by the spring 181. Therefore, an impact force in the direction of insertion, which occurs when the insertion-side connector body 155 is engaged in the engaging portion 171, can be absorbed by the spring 181, and at the same time the optical lens 164 and the optical lens 116 can be put in close contact.

According to the present embodiment, the inner peripheral wall portion 169 of the terminal fixing member 167 of the power unit 154 is provided with the first wiper 176 and second wiper 177 before and behind the electrical terminal portion 97. Thereby, in accordance with insertion/removal of the insertion-side connector body 155 in/from the terminal fixing member 167 of the power unit 154, moisture adhering to the surface of the inner peripheral wall portion 169 of the terminal fixing member 167 can be removed by the first wiper 176. Furthermore, the second wiper 177 can prevent moisture, which adheres to the distal end side of the inner peripheral wall portion 169 of the terminal fixing member 167, from flowing toward the electrical contact portion 59 by force of gravity. Therefore, even if moisture adheres to the electrical contact portion 59 when the power unit 154 and the insertion-section-side body 153 are connected, the moisture on the electrical contact portion 59 can be removed in the process of connecting the power unit 154 and the insertion-section-side body 153. Moreover, there is no concern that moisture that is removed or moisture that is not removed and remains on the connector section adheres to the electrical contact portion 59, and the reliability can be improved.

The present invention is not limited directly to the above-described embodiments. In practice, the structural elements can be modified and embodied without departing from the point of the invention. Various inventions can be made by properly combining the structural elements disclosed in the embodiments. For example, some structural elements may be omitted from all the structural elements disclosed in the embodiments. Furthermore, structural elements in different embodiments may properly be combined.

## Claims

1. A medical apparatus comprising:
a first connector portion (51) including an insertion hole portion (86) and a ring-shaped terminal juxtaposition portion on which a plurality of terminals (97) are juxtaposed along an inner peripheral surface of the insertion hole portion (86);
a second connector portion (50) including
a cylindrical insertion-side connector body (69) having an outer peripheral surface and a ring-shaped terminal juxtaposition portion on which a plurality of electrical contact portions (59) are juxtaposed in a circumferential direction on said outer peripheral surface of the cylindrical insertion-side connector body (69),
wherein said plurality of electrical contact portions (59) of said second connector portion (50) are configured to be connected to the plurality of terminals (97) of the first connector portion (51) when said cylindrical insertion-side connector body (69) of said second connector portion (50) is inserted into said insertion hole portion (86) of said first connector portion (51);
a removing member (101) provided in the first connector portion (51) and including a sliding contact portion (101b) which is configured to remove moisture on the plurality of electrical contact portions (59) by coming into sliding contact with the outer peripheral surface of said cylindrical insertion-side connector body (69) when the plurality of terminals (97) of said first connector portion (51) are connected to the plurality of electrical contact portions (59) of said second connector portion (50), a distal end portion of the outer peripheral surface of said cylindrical insertion-side connector body (69) being configured to first come into contact with the sliding contact portion (101b) when said cylindrical insertion-side connector body (69) of said second connector portion (50) is inserted into said insertion hole portion (86) of said first connector portion (51); and
a prevention member (102) including a sliding contact portion (102b) configured to come into sliding contact with the outer peripheral surface of said cylindrical insertion-side connector body (69) of the second connector portion (50) and to thereby be bent and brought into close contact with the outer peripheral surface of the cylindrical insertion-side connector body (69) to shut off the distal end portion of the outer peripheral surface of said cylindrical insertion-side connector body (69) from a distal end side so that the prevention member (102) can prevent moisture from flowing from the distal end side toward the plurality of electrical contact portions (59), wherein
each of the removing member (101) and the prevention member (102) is an annular member including a fixing portion which is fixed on the inner peripheral surface of the insertion hole portion (86) of the first connector portion (51).

2. A medical apparatus comprising:
a first connector portion (154) including an insertion portion (169) and a ring-shaped terminal juxtaposition portion on which a plurality of terminals (97) are juxtaposed along an outer peripheral surface of the insertion portion (169);
a second connector portion (153) including
a cylindrical insertion-side connector body (155) having an inner peripheral surface and a ring-shaped terminal juxtaposition portion on which a plurality of electrical contact portions (59) are juxtaposed in a circumferential direction on said inner peripheral surface of the cylindrical insertion-side connector body (69),
wherein said plurality of electrical contact portions (59) of said second connector portion (153) are configured to be connected to the plurality of terminals (97) of the first connector portion (154) when said insertion portion (169) of said first connector portion (154) is inserted into said cylindrical insertion-side connector body (155) of said second connector portion (153);
a removing member (176) provided in the first connector portion (154) and including a sliding contact portion which is configured to remove moisture on the plurality of electrical contact portions (59) by coming into sliding contact with the inner peripheral surface of said cylindrical insertion-side connector body (155) when the plurality of terminals (97) of said first connector portion (154) are connected to the plurality of contact portions (59) of said second connector portion (153), a distal end portion of the inner peripheral surface of the insertion-side connector body (155) being configured to first come into contact with the sliding contact portion when said insertion portion (169) of said first connector portion (154) is inserted into said cylindrical insertion-side connector body (155) of said second connector portion (153); and
a prevention member (177) including a sliding contact portion configured to come into sliding contact with the inner peripheral surface of said cylindrical insertion-side connector body (155) of the second connector portion (153) and to thereby be bent and brought into close contact with the inner peripheral surface of the cylindrical insertion-side connector body (155) to shut off the distal end portion of the inner peripheral surface of said cylindrical insertion-side connector body (155) from a distal end side so that the prevention member (177) can prevent moisture from flowing from the distal end side toward the plurality of electrical contact portions (59), wherein
each of the removing member (176) and the prevention member (177) is an annular member including a fixing portion which is fixed on the outer peripheral surface of the insertion portion (169) of the first connector portion (154).

3. The medical apparatus of claim 1 or 2, wherein the inner diameter of the sliding contact portion (102b) of the prevention member (102) is slightly less than the outside diameter of the cylindrical insertion-side connector body (69; 155).

4. The medical apparatus according to claim 1 or 2, wherein the removing member (101; 176) is an elastic member.

5. The medical apparatus according to claim 1 or 2, wherein the prevention member (102; 177) is an elastic member.

6. The medical apparatus according to claim 1 or 2, wherein
the medical apparatus includes a motor-driven bending endoscope (21) which bend-operates a bending section (25) by motor driving,
the motor-driven bending endoscope (21) includes an insertion-section-side body (50; 153) and a power unit (51; 154) to which the insertion-section-side body (50; 153) is detachably connected,
the first connector portion (51; 154) is provided on the power unit (51; 154), and
the second connector portion (50; 153) is provided on the insertion-section-side body (50; 153).

7. The medical apparatus according to claim 6, wherein
the insertion-section-side body (50; 153) includes a signal processing member which is connected to the plurality of electrical contact portions (59), and
the power unit (51; 154) includes a signal cable which is connected to the plurality of terminals (97) for electrical connection.

8. The medical apparatus according to claim 7, wherein the power unit (51; 154) is configured such that the removing member (101; 176) and the prevention member (102; 177) are disposed spaced apart, with the plurality of terminals (97) being interposed.

## Patentansprüche

1. Medizinisches Gerät, das umfasst:
einen ersten Verbinderabschnitt (51) mit einem Einführöffnungsabschnitt (86) und einem ringförmigen Anschluss-Nebeneinanderpositionierungs-Abschnitt, an dem eine Mehrzahl von Anschlüssen (97) entlang einer Innenumfangsfläche des Einführöffnungsabschnitts (86) nebeneinander angeordnet ist;
einen zweiten Verbinderabschnitt (50) mit einem zylindrischen Einführseitenverbinderkörper (69), der eine Außenumfangsfläche und einen ringförmigen Anschluss-Nebeneinanderpositionierungs-Abschnitt hat, an dem eine Mehrzahl von elektrischen Kontaktabschnitten (59) in einer Umfangsrichtung an der Außenumfangsfläche des zylindrischen Einführseitenverbinderkörpers (69) nebeneinander angeordnet ist,
wobei die Mehrzahl von elektrischen Kontaktabschnitten (59) des zweiten Verbinderabschnitts (50) dazu eingerichtet ist, mit der Mehrzahl von Anschlüssen (97) des ersten Verbinderabschnitts (51) verbunden zu werden, wenn der zylindrische Einführseitenverbinderkörper (69) des zweiten Verbinderabschnitts (50) in den Einführöffnungsabschnitt (86) des ersten Verbinderabschnitts (51) eingeführt wird;
ein Entfernungselement (101), das in dem ersten Verbinderabschnitt (51) vorgesehen ist und einen Gleitkontaktabschnitt (101b) umfasst, der dazu eingerichtet ist, Feuchtigkeit auf der Mehrzahl von elektrischen Kontaktabschnitten (59) zu entfernen, indem er mit der Außenumfangsfläche des zylindrischen Einführseitenverbinderkörpers (69) in Gleitkontakt kommt, wenn die Mehrzahl von Anschlüssen (97) des ersten Verbinderabschnitts (51) mit der Mehrzahl von elektrischen Kontaktabschnitten (69) des zweiten Verbinderabschnitts (50) verbunden werden, wobei ein distaler Endabschnitt der Außenumfangsfläche des zylindrischen Einführseitenverbinderkörpers (69) dazu eingerichtet ist, zuerst mit dem Gleitkontaktabschnitt (101b) in Kontakt zu kommen, wenn der zylindrische Einführseitenverbinderkörper (69) des zweiten Verbinderabschnitts (50) in den Einführöffnungsabschnitt (86) des ersten Verbinderabschnitts (51) eingeführt wird;
ein Verhinderungselement (102) mit einem Gleitkontaktabschnitt (102b), der dazu eingerichtet ist, in Gleitkontakt mit der Außenumfangsfläche des zylindrischen Einführseitenverbinderkörpers (69) des zweiten Verbinderabschnitts (50) zu kommen und dadurch gebogen und in engen Kontakt mit der Außenumfangsfläche des zylindrischen Einführseitenverbinderkörpers (69) gebracht zu werden, um den distalen Endabschnitt der Außenumfangsfläche des zylindrischen Einführseitenverbinderkörpers (69) von einer distalen Endseite abzusperren, so dass das Verhinderungselement (102) verhindern kann, dass Feuchtigkeit von der distalen Endseite in Richtung der Mehrzahl von elektrischen Kontaktabschnitten (59) fließt, wobei
das Entfernungselement (101) und das Verhinderungselement (102) jeweils ein ringförmiges Element mit einem Befestigungsabschnitt ist, der an der Innenumfangsfläche des Einführöffnungsabschnitts (86) des ersten Verbinderabschnitts (51) befestigt ist.

2. Medizinisches Gerät, das umfasst:
einen ersten Verbinderabschnitt (154) mit einem Einführabschnitt (169) und einem ringförmigen Anschluss-Nebeneinanderpositionierungs-Abschnitt, an dem eine Mehrzahl von Anschlüssen (97) entlang einer Innenumfangsfläche des Einführabschnitts (169) nebeneinander angeordnet ist;
einen zweiten Verbinderabschnitt (153) mit einem zylindrischen Einführseitenverbinderkörper (155), der eine Innenumfangsfläche und einen ringförmigen Anschluss-Nebeneinanderpositionierungs-Abschnitt hat, an dem eine Mehrzahl von elektrischen Kontaktabschnitten (59) in einer Umfangsrichtung an der Innenumfangsfläche des zylindrischen Einführseitenverbinderkörpers (69) nebeneinander angeordnet ist,
wobei die Mehrzahl von elektrischen Kontaktabschnitten (59) des zweiten Verbinderabschnitts (153) dazu eingerichtet ist, mit der Mehrzahl von Anschlüssen (97) des ersten Verbinderabschnitts (154) verbunden zu werden, wenn der Einführabschnitt (169) des ersten Verbinderabschnitts (154) in den zylindrischen Einführseitenverbinderkörper (155) des zweiten Verbinderabschnitts (153) eingeführt wird;
ein Entfernungselement (176), das in dem ersten Verbinderabschnitt (154) vorgesehen ist und einen Gleitkontaktabschnitt umfasst, der dazu eingerichtet ist, Feuchtigkeit auf der Mehrzahl von elektrischen Kontaktabschnitten (59) zu entfernen, indem er mit der Innenumfangsfläche des zylindrischen Einführseitenverbinderkörpers (155) in Gleitkontakt kommt, wenn die Mehrzahl von Anschlüssen (97) des ersten Verbinderabschnitts (154) mit der Mehrzahl von Kontaktabschnitten (59) des zweiten Verbinderabschnitts (153) verbunden werden, wobei ein distaler Endabschnitt der Innenumfangsfläche des zylindrischen Einführseitenverbinderkörpers (155) dazu eingerichtet ist, zuerst mit dem Gleitkontaktabschnitt in Kontakt zu kommen, wenn der Einführabschnitt (169) des ersten Verbinderabschnitts (154) in den zylindrischen Einführseitenverbinderkörper (155) des zweiten Verbinderabschnitts (153) eingeführt wird;
ein Verhinderungselement (177) mit einem Gleitkontaktabschnitt, der dazu eingerichtet ist, in Gleitkontakt mit der Innenumfangsfläche des zylindrischen Einführseitenverbinderkörpers (155) des zweiten Verbinderabschnitts (153) zu kommen und dadurch gebogen und in engen Kontakt mit der Innenumfangsfläche des zylindrischen Einführseitenverbinderkörpers (155) gebracht zu werden, um den distalen Endabschnitt der Innenumfangsfläche des zylindrischen Einführseitenverbinderkörpers (155) von einer distalen Endseite abzusperren, so dass das Verhinderungselement (177) verhindern kann, dass Feuchtigkeit von der distalen Endseite in Richtung der Mehrzahl von elektrischen Kontaktabschnitten (59) fließt, wobei
das Entfernungselement (176) und das Verhinderungselement (177) jeweils ein ringförmiges Element mit einem Befestigungsabschnitt ist, der an der Außenumfangsfläche des Einführabschnitts (169) des ersten Verbinderabschnitts (154) befestigt ist.

3. Medizinisches Gerät nach Anspruch 1 oder 2, bei dem der Innendurchmesser des Gleitkontaktabschnitts (102b) des Verhinderungselements (102) geringfügig geringer ist als der Außendurchmesser des zylindrischen Einführseitenverbinderkörpers (69; 155).

4. Medizinisches Gerät nach Anspruch 1 oder 2, bei dem das Entfernungselement (101; 176) ein elastisches Element ist.

5. Medizinisches Gerät nach Anspruch 1 oder 2, bei dem das Verhinderungselement (102; 177) ein elastisches Element ist.

6. Medizinisches Gerät nach Anspruch 1 oder 2, bei dem
das medizinische Gerät ein von einem Motor angetriebenes Biegeendoskop (21) umfasst, das einen Biegeabschnitt (25) durch einen Motorantrieb biegebetätigt,
wobei das von einem Motor angetriebene Biegeendoskop (21) einen Einführabschnittsseitenkörper (50; 153) und eine Energieeinheit (51; 154) umfasst, mit der der Einführabschnittsseitenkörper (50; 103 50) lösbar verbunden ist,
wobei der erste Verbinderabschnitt (51; 154) an der Energieeinheit (51; 154) vorgesehen ist und
der zweite Verbinderabschnitt (50; 153) an dem Einführabschnittsseitenkörper (50; 153) vorgesehen ist.

7. Medizinisches Gerät nach Anspruch 6, bei dem
der Einführabschnittsseitenkörper (50; 153) ein Signalverarbeitungselement umfasst, das mit der Mehrzahl von elektrischen Kontaktabschnitten (59) verbunden ist, und
die Energieeinheit (51; 154) ein Signalkabel umfasst, das mit der Mehrzahl von Anschlüssen (97) für eine elektrische Verbindung verbunden ist.

8. Medizinisches Gerät nach Anspruch 7, bei dem die Energieeinheit (51; 154) so gestaltet ist, dass das Entfernungselement (101; 176) und das Verhinderungselement (102; 177) voneinander beabstandet angeordnet sind, wobei die Mehrzahl von Anschlüssen (97) dazwischen angeordnet ist.

## Revendications

1. Appareil médical comprenant :
une première partie (51) de connecteur incluant une partie (86) de trou d'insertion et une partie de juxtaposition de bornes en forme de bague sur laquelle une pluralité de bornes (97) sont juxtaposées le long d'une surface périphérique intérieure de la partie (86) de trou d'insertion ;
une deuxième partie (50) de connecteur incluant un corps (69) cylindrique de connecteur de côté insertion ayant une surface périphérique extérieure et une partie de juxtaposition de bornes en forme de bague sur laquelle une pluralité de parties (59) de contact électrique sont juxtaposées dans un sens circonférentiel sur ladite surface périphérique extérieure du corps (69) cylindrique de connecteur de côté insertion,
dans lequel ladite pluralité de parties (59) de contact électrique de ladite deuxième partie (50) de connecteur sont configurées pour être connectées à la pluralité de bornes (97) de la première partie (51) de connecteur lorsque ledit corps (69) cylindrique de connecteur de côté insertion de ladite deuxième partie (50) de connecteur est inséré dans ladite partie (86) de trou d'insertion de ladite première partie (51) de connecteur ;
un élément (101) d'élimination prévu dans la première partie (51) de connecteur et incluant une partie (101b) de contact coulissant qui est configuré pour éliminer l'humidité sur la pluralité de parties (59) de contact électrique en venant en contact coulissant avec la surface périphérique extérieure dudit corps (69) cylindrique de connecteur de côté insertion lorsque la pluralité de bornes (97) de ladite première partie (51) de connecteur sont connectées à la pluralité de parties (59) de contact électrique de ladite deuxième partie (50) de connecteur, une partie d'extrémité distale de la surface périphérique extérieure dudit corps (69) cylindrique de connecteur de côté insertion étant configurée pour venir d'abord en contact avec la partie (101b) de contact coulissant lorsque ledit corps (69) cylindrique de connecteur de côté insertion de ladite deuxième partie (50) de connecteur est inséré dans ladite partie (86) de trou d'insertion de ladite première partie (51) de connecteur ;
un élément (102) de prévention incluant une partie (102b) de contact coulissant configuré pour venir en contact coulissant avec la surface périphérique extérieure dudit corps (69) cylindrique de connecteur de côté insertion de la deuxième partie (50) de connecteur et pour ainsi être courbé et amené en contact étroit avec la surface périphérique extérieure du corps (69) cylindrique de connecteur de côté insertion pour fermer la partie d'extrémité distale de la surface périphérique extérieure du corps (69) cylindrique de connecteur de côté insertion depuis un côté d'extrémité distale de telle sorte que l'élément (102) de prévention peut empêcher l'humidité de s'écouler du côté d'extrémité distale vers la pluralité de parties (59) de contact électrique, dans lequel
chacun de l'élément (101) d'élimination et de l'élément (102) de prévention est un élément annulaire incluant une partie de fixation qui est fixée sur la surface périphérique intérieure de la partie (86) de trou d'insertion de la première partie (51) de connecteur.

2. Appareil médical comprenant :
une première partie (154) de connecteur incluant une partie (169) d'insertion et une partie de juxtaposition de bornes en forme de bague sur laquelle une pluralité de bornes (97) sont juxtaposées le long d'une surface périphérique extérieure de la partie (169) d'insertion ;
une deuxième partie (153) de connecteur incluant un corps (155) cylindrique de connecteur de côté insertion ayant une surface périphérique intérieure et une partie de juxtaposition de bornes en forme de bague sur laquelle une pluralité de parties (59) de contact électrique sont juxtaposées dans un sens circonférentiel sur ladite surface périphérique intérieure du corps (69) cylindrique de connecteur de côté insertion,
dans lequel ladite pluralité de parties (59) de contact électrique de ladite deuxième partie (153) de connecteur sont configurées pour être connectées à la pluralité de bornes (97) de la première partie (154) de connecteur lorsque ladite partie (169) d'insertion de ladite première partie (154) de connecteur est insérée dans ledit corps (155) cylindrique de connecteur de côté insertion de ladite deuxième partie (153) de connecteur ;
un élément (176) d'élimination prévu dans la première partie (154) de connecteur et incluant une partie de contact coulissant qui est configuré pour éliminer l'humidité sur la pluralité de parties (59) de contact électrique en venant en contact coulissant avec la surface périphérique intérieure dudit corps (155) cylindrique de connecteur de côté insertion lorsque la pluralité de bornes (97) de ladite première partie (154) de connecteur sont connectées à la pluralité de parties (59) de contact électrique de ladite deuxième partie (153) de connecteur, une partie d'extrémité distale de la surface périphérique intérieure du corps (155) de connecteur de côté insertion étant configurée pour venir d'abord en contact avec la partie de contact coulissant lorsque ladite partie (169) d'insertion de ladite première partie (154) de connecteur est insérée dans ledit corps (155) cylindrique de connecteur de côté insertion de ladite deuxième partie (153) de connecteur ;
un élément (177) de prévention incluant une partie de contact coulissant configuré pour venir en contact coulissant avec la surface périphérique intérieure dudit corps (155) cylindrique de connecteur de côté insertion de la deuxième partie (153) de connecteur et pour ainsi être courbé et amené en contact étroit avec la surface périphérique intérieure du corps (155) cylindrique de connecteur de côté insertion pour fermer la partie d'extrémité distale de la surface périphérique intérieure dudit corps (155) cylindrique de connecteur de côté insertion depuis un côté d'extrémité distale de telle sorte que l'élément (177) de prévention peut empêcher l'humidité de s'écouler du côté d'extrémité distale vers la pluralité de parties (59) de contact électrique, dans lequel
chacun de l'élément (176) d'élimination et de l'élément (177) de prévention est un élément annulaire incluant une partie de fixation qui est fixée sur la surface périphérique extérieure de la partie (169) d'insertion de la première partie (154) de connecteur.

3. Appareil médical selon la revendication 1 ou 2, dans lequel le diamètre intérieur de la partie (102b) de contact coulissant de l'élément (102) de prévention est légèrement inférieur au diamètre extérieur du corps (69 ; 155) cylindrique de connecteur de côté insertion.

4. Appareil médical selon la revendication 1 ou 2, dans lequel l'élément (101 ; 176) d'élimination est un élément élastique.

5. Appareil médical selon la revendication 1 ou 2, dans lequel l'élément (102 ; 177) de prévention est un élément élastique.

6. Appareil médical selon la revendication 1 ou 2, dans lequel
l'appareil médical inclut un endoscope (21) flexible motorisé qui opère en flexion une section de flexion (25) par entraînement motorisé,
l'endoscope (21) flexible motorisé inclut un corps (50 ; 153) de côté de section d'insertion et une unité (51 ; 154) d'alimentation électrique à laquelle le corps (50 ; 153) de côté de section d'insertion est connecté de façon détachable,
la première partie (51 ; 154) de connecteur est prévue sur l'unité (51 ; 154) d'alimentation électrique, et
la deuxième partie (50 ; 153) de connecteur est prévue sur le corps (50 ; 153) de côté de section d'insertion.

7. Appareil médical selon la revendication 6, dans lequel
le corps (50 ; 153) de côté de section d'insertion inclut un élément de traitement de signaux qui est connecté à la pluralité de parties (59) de contact électrique, et
l'unité (51 ; 154) d'alimentation électrique inclut un câble de signaux qui est connecté à la pluralité de bornes (97) pour connexion électrique.

8. Appareil médical selon la revendication 7, dans lequel l'unité (51 ; 154) d'alimentation électrique est configurée de telle manière que l'élément (101 ; 176) d'élimination et l'élément (102 ; 177) de prévention sont disposés espacés, avec la pluralité de bornes (97) étant interposées.
